(19) Europäisches Patentamt
European Patent Office
Office européen des brevets

(11) Publication number: **0 231 045 B1**

(12) **EUROPEAN PATENT SPECIFICATION**

(45) Date of publication of patent specification: **27.05.92** (51) Int. Cl.[5]: **C07C 271/06**

(21) Application number: **87200069.0**

(22) Date of filing: **16.01.87**

(54) **A process for the preparation of carbamates.**

(30) Priority: **28.01.86 GB 8601962**

(43) Date of publication of application:
**05.08.87 Bulletin 87/32**

(45) Publication of the grant of the patent:
**27.05.92 Bulletin 92/22**

(84) Designated Contracting States:
**BE DE FR GB NL**

(56) References cited:
**EP-A- 0 086 281** **EP-A- 0 129 759**
**EP-A- 0 169 650** **EP-A- 0 224 292**
**DE-A- 2 555 557** **US-A- 4 331 560**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031CM Amsterdam(NL)**

**EP 0 231 045 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of carbamates and to carbamates prepared by means of such process.

It is known from US Patent Specification 3,448,140 to prepare a carbamate by reaction of an aromatic nitro compound with a hydroxyl group containing compound and carbon monoxide with the aid of a catalyst. Specifically it is known to prepare methyl carbanilate from nitrobenzene, methanol and carbon monoxide in the presence of a palladium compound and ferrichloride. As a palladium compound may be used palladium dichloro bis-triphenylphosphine. However the yield of the carbamate, viz. methyl carbanilate is still moderate, about 66%.

It has now been found that with specific palladium catalyst compositions increased yields can be obtained under mild reaction conditions, even as high as 95%.

Accordingly, the present invention provides a process for the preparation of carbamates which comprises reacting an aromatic nitro compound with a hydroxyl group containing organic compound and carbon monoxide in the presence of a catalytic system comprising

a) palladium and/or a palladium compound

b) a ligand having the formula I

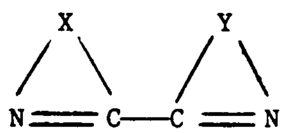

(I)

in which X and Y represent the same or different bridging groups each of which has 3 or 4 atoms in the bridge, of which atoms at least two are carbon atoms and which groups X and Y may be bound to each other by means of a connection other than that already formed by the carbon atoms shown in formula I and

c) a compound MZ, wherein M is a metal of the group consisting of Cu, Fe, V, Cr, Zn, Sn, U and Ce and Z is an anion of an acid having a pK of less than 2, except of a hydrohalogenic acid.

In European Patent Application (Publication) 86281 is disclosed a process for the preparation of carbamates by reacting an aromatic nitro compound with a hydroxyl group containing organic compound and carbon monoxide in the presence of a catalytic system comprising palladium and/or a palladium compound, a ligand, such as 2,2'-bipyridine or 1,10-phenanthroline and an acid having a $pK_a$ of less than 3.5, e.g. p-toluene sulphonic acid or trifluoromethyl sulphonic acid. European Patent Application 86281 does not reveal that specific salts of the strong acids could be suitably used in the catalytic system.

The process according to the invention is carried out in the presence of palladium or a palladium compound. Palladium can be used as metal, deposited on an inert carrier, such as carbon or alumina, or in the form of palladium compounds, especially palladium salts. Excellent results are obtained when the palladium compound is soluble in the reaction mixture.

Examples of palladium salts include palladium chloride, palladium bromide, palladium iodide, sodium tetrachloro palladate, potassium tetrachloropalladate, potassium tetraiodopalladate, palladium carboxylates, such as palladium acetate, palladium propionate, palladium isobutyrate and palladium acetylacetonate. Preference is given to the use of palladium salts of organic acids, in particular of an alkanoic acid having not more than 12 carbon atoms per molecule. Most preferred is palladium diacetate.

The bridging groups X and Y in the formula of the ligand are connected with each other via the two carbon atoms as shown. Apart from this connection there may exist a second connection between the bridging groups, as is the case in 1,10-phenanthroline (also called 1,10-diazaphenanthrene) and derivatives thereof. Any atoms in the bridging groups X and Y other than carbon atoms are preferably nitrogen atoms. Furthermore, X and Y are preferably the same.

Examples of suitable bidentate ligands of formula I are 2,2'-bipyridyl and derivatives thereof, for example 4,4'-dimethyl-2,2'-bipyridyl, 4,4'-dichloro-2,2'-bipyridyl, 4,4'-dimethoxy-2,2'-bipyridyl, 4,4'-dicarboxy-2,2'-bipyridyl and 2,2'-biquinolyl.

Other examples of suitable bidentate ligands of formula I are 1,10-phenanthroline and derivatives thereof, for example 5-chlorophenanthroline, 4,7-diphenylphenanthroline, 4,7-dimethylphenanthroline, 2,9-dichlorophenanthroline, 1,10-phenanthroline-5-sulphonic acid and 4,7-diphenyl-1,10-phenanthroline-disulphonic acids.

Further examples of suitable bidentate ligands are 2-(2-pyridyl)benzimidazole, 3-(2-pyridyl)-5,6-diphenyl-1,2,4-triazine and the monosodium salt of 3-(2-pyridyl)-5,6-diphenyl-1,2,4-triazine-p,p′-disulphonic acid.

Preferred ligands are 2,2′-bipyridyl or a derivative thereof or 1,10-phenanthroline or a derivative thereof. Particularly preferred are 2,2′-bipyridyl and 1,10-phenanthroline.

The third component of the catalytic system is the compound of the formula MZ, wherein M represents a metal of the group consisting of copper, iron, vanadium, chromium, zinc, tin, uranium and cerium. More preferred are copper, iron, vanadium, chromium and uranium. Z represents an anion of an acid having a pK of less than 2, except the anion of hydrohalogenic acids. Preferred are the anions of the strong acids $H_2SO_4$, $HBF_4$, p-toluene sulphonic acid or alkyl-substituted derivatives of the latter or $HClO_4$. Other suitable acids are for example benzene sulphonic acid, naphthalene sulphonic acid, 2,4,5-trichlorobenzene sulphonic acid, or corresponding bromo- and fluoro-derivatives.

Other examples of acids are those, that can be formed, by interaction of a Lewis acid, such as $BF_3$, $AsF_5$, $SbF_5$, $PF_5$, $TaF_5$ or $NbF_5$ with a Broensted acid, such as a hydrohalogenic acid, e.g. HF, fluorosulphonic acid, phosphoric acid or sulphuric acid. Specific examples are $H_2SiF_6$, $HBF_4$, $HPF_6$ and $HSbF_6$. Examples of sulphonic acids are fluorosulphonic acid, trifluoromethylsulphonic acid and chlorosulphonic acid.

As specific examples of the compound MZ may be mentioned copper(1)tosylate, copper(2)tosylate, copper(2) chlorate, iron(2) chlorate, copper(2) fluoroborate, tin(4) sulphate, uranium(4) sulphate.

As stated hereinbefore, the carbamates are prepared from aromatic nitro compounds, i.e. compounds containing at least one aromatic group in which an $NO_2$ group is directly attached to a carbon atom forming part of the aromatic nucleus. Examples of aromatic nitro compounds include nitrobenzene, alkyl- and alkoxy substituted nitrobenzenes, aryl- and aryloxy substituted nitrobenzenes, dinitrobenzenes, alkyl-, alkoxy-, aryl- and aryloxy substituted dinitrobenzenes, such as 2,4-dinitrotoluene, 2,6-dinitrotoluene. Further may be mentioned 4,4′-dinitrodiphenylmethane and polynitrobenzenes. Preferred aromatic nitro compounds are nitrobenzene, m-dinitrobenzene, 2-,3-and 4-nitrotoluene, 2,4-dinitrotoluene, 2,6-dinitrotoluene and 4,4′-dinitrodiphenylmethane.

The process according to the present invention is carried out by reacting an aromatic nitro compound with carbon monoxide and an organic compound containing at least one hydroxyl group. Mono- or polyhydric alcohols containing primary, secondary or tertiary hydroxyl groups as well as mixtures of such compounds can be used. The organic compounds containing at least one hydroxyl group can be represented by the general formula $R(OH)_m$ wherein m is an integer up to 4 and R represents a substituted or unsubstituted alkyl, aryl, alkaryl or aralkyl group containing up to 20, preferably up to 6 carbon atoms.

Examples of compounds according to the general formula $R(OH)_m$ wherein m and R are as defined hereinbefore comprise mono-hydric alcohols such as methanol, ethanol, n-propanol, sec-propanol, the butanols, amyl alcohol, hexyl alcohol, lauryl alcohol, cetyl alcohol, benyl alcohol, chlorobenzyl alcohol, methoxy benzyl alcohol, methoxy ethanol, butoxy ethanol, cyclohexyl alcohol, phenol and the cresols. Examples of polyhydric alcohols comprise diols, e.g. ethylene glycol, diethylene glycol, propylene glycol, dipropylene glycol and triols such as glycerol, trimethylolpropane and the hexane triols. Ethers of the polyhydric compounds can also be used provided that they contain at least one free hydroxyl group in the molecule. Preference is given to the use of lower alcohols such as methanol, ethanol, propanol, ispropanol, butanol, sec-butanol, isobutanol, ethylene glycol, glycerol and trimethylolpropane, in particular to methanol and ethanol.

The basic reaction equation may be written as follows

$$C_6H_5NO_2 + 3CO + CH_3OH \longrightarrow C_6H_5-\underset{\underset{H}{|}}{N}-\overset{\overset{O}{\|}}{C}-OCH_3 + 2CO_2.$$

The amount of palladium or palladium compound to be used in the process according to the invention, is conveniently between 0.001 %w, and 10 %w, in particular between 0.01 %w and 2 %w, calculated on the amount of aromatic nitro compound. Amounts between 0.1 %w and 0.5 %w are most preferred.

The ligand is generally present in such an amount that the mol ratio of ligand:palladium (compound) is between 20 and 0.5.

The amount of compound MZ may range between 0,1 and 100 mol per gramatom of palladium.

The process according to the invention may be carried out conveniently at temperatures up to 300 °C. Preference is given to the use of temperatures in the range of from 75 °C to 200 °C. The reaction is

normally carried out at superatmospheric pressure; pressures of up to 500 bar may be applied. Preferably the reaction is carried out at a pressure in the range of from 10 to 150 bar.

Apart from the three above-mentioned catalyst components in the catalyst composition, it may also comprise a fourth component, namely a free protonic acid, except a hydrohalogenic acid. Generally the same acids from which the anion Z was present can be used, but also acids with a pK higher than 2 may be used, such as alkanoic acids. These alkanoic acids preferably have no more than 12 carbon atoms per molecule, for example acetic acid, propionic acid, butyric acid, isobutyric acid, valeric acid, pivalic acid, heptanoic acid and lauric acid.

The process according to the present invention is suitably carried out in the presence of an aprotic solvent. Examples of suitable solvents are hydrocarbons, such as hexane, cyclohexane, octane, benzene, toluene, o-xylene, m-xylene, p-xylene, ethylbenzene, cumene; halogenated hydrocarbons, such as chloroform, 1,2-dichloroethane, perfluoroalkanes, chlorobenzene and the three dichlorobenzenes; ethers, such as diethyl ether, tetrahydrofuran, 3,6-dioxaoctane, methyl tert.-butyl ether, dioxane, anisole, 2,5,8-trioxanonane, diphenyl ether and diisopropyl ether; N,N-dialkyl substituted amides, such as N,N-dimethylformamide and N-methylpyrrolidone; sulphones such as diisopropyl sulphone and tetrahydrothiophene 1,1-dioxide (also referred to as "sulfolane"); esters, such as methyl benzoate and ethyl acetate.

The process according to the present invention can be carried out batchwise, semi-continuously or continuously. The reaction time is, of course, related to the temperature and pressure adopted. In general reaction times between 1 and 20 hours are adequate.

The carbamates produced according to the process of the present invention can be used as starting materials for agrochemicals, dyes, pharmaceuticals or polyurethanes, or may be converted into the corresponding isocyanates by methods known in the art, e.g. by heating the appropriate carbamate.

## Examples

A 300 ml autoclave (Hastalloy C) was charged with nitrobenzene (10 ml), methanol (50 ml), palladium diacetate (0.1 mmol), 1,10 phenanthroline (10 mmol) and copper (2) tosylate (1 mmol). The autoclave was then pressurized with carbon monoxide (70 bar). The temperature was raised to 135 °C and the reaction mixture was kept at this temperature and a pressure of 70 bar during 2 hours. The reaction mixture was allowed to cool and was analysed thereafter by gas-liquid chromatography. The conversion of nitrobenzene amounted to 100%, and the selectivity to methylcarbanilate was 95%.

A number of further experiments under the same conditions as above were conducted with catalysts of different composition.

The results are given in the following table.

All numbers under catalyst composition express mmol of the components.

| EX. | CATALYST COMPOSITION | REACTION TIME h | TEMP. °C | CONVERSION of nitrobenzene | SELECTIVITY to CARBAMATE (methylcarbanilate) |
|---|---|---|---|---|---|
| 1 | 0.1 Pd $(OAc)_2$<br>10 PHENANTHROLINE<br>1 Cu $(tosylate)_2$ | 2 | 135 | 100 | 95 |
| 2 | 0.1 Pd $(OAc)_2$<br>10 PHENANTHROLINE<br>1 Cu $(ClO_4)_2$ | 2 | 135 | 100 | 95 |
| 3 | 0.1 Pd $(OAc)_2$<br>10 PHENANTHROLINE<br>1 Fe $(ClO_4)_2$ | 2 | 135 | 97 | 95 |
| 4 | 0.1 Pd $(OAc)_2$<br>5 PHENANTHROLINE<br>1 $VOSO_4$ | 2 | 135 | 100 | 98 |
| 5 | 0.1 Pd $(OAc)_2$<br>5 PHENANTHROLINE<br>1 $Cr_2SO_4$ | 5 | 135 | 100 | 92 |
| 6 | 0.1 $Pd(OAc)_2$<br>5 PHENANTHROLINE<br>1 $U(SO_4)_2$ | 5 | 135 | 100 | 91 |
| 7 | 0.1 Pd $(OAc)_2$<br>5 PHENANTHROLINE<br>0.5 Sn $(SO_4)_2$ | 5 | 135 | 99 | 92 |
| 8 | 0.1 Pd $(OAc)_2$<br>10 PHENANTHROLINE<br>1 Cu $(OAc)_2$<br>(COMPARATIVE) | 5 | 135 | 95 | 30 |

## Claims

**1.** A process for the preparation of carbamates which comprises reacting an aromatic nitro compound with a hydroxyl group containing organic compound and carbon monoxide in the presence of a catalytic system comprising

a) palladium and/or a palladium compound

b) a ligand having the formula I

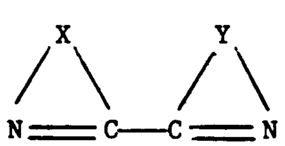

(I)

in which X and Y represent the same or different bridging groups each of which has 3 or 4 atoms in the bridge, of which atoms at least two are carbon atoms and which groups X and Y may be bound to each other by means of a connection other than that already formed by the carbon atoms shown in formula I and

c) a compound MZ, wherein M is a metal of the group consisting of Cu, Fe, V, Cr, Zn, Sn, U and Ce and Z is an anion of an acid having a pK of less than 2, except of a hydrohalogenic acid.

**2.** A process according to claim 1 in which the ligand is 1,10-phenanthroline or a derivative thereof.

3. A process according to claim 1 in which the ligand is 2,2′-bipyridyl or a derivative thereof.

4. A process according to claims 1-3 in which the palladium catalyst is a homogeneous catalyst.

5. A process according to claim 4 in which the catalyst is a palladium salt.

6. A process according to claim 5 in which the catalyst is palladium diacetate.

7. A process according to claims 1-6 in which Z is an anion of $H_2SO_4$, $HBF_4$, p-toluene sulphonic acid or alkyl substituted derivative of the latter, or $HClO_4$.

8. A process according to claim 7 in which copper (2) tosylate, copper (2) chlorate, iron (2) chlorate, copper (2) fluoroborate, zinc fluoroborate, tin (4) sulphate or uranium (4) sulphate is used.

9. A process according to any one of the preceding claims in which the reaction is carried out at a temperature in the range of from 75 to 200 °C.

10. A process according to any one of the preceding claims in which the reaction is carried out at a pressure in the range of from 10 to 150 bar.

## Revendications

1. Procédé de préparation de carbamates, qui consiste à faire réagir un composé nitro aromatique avec un composé organique contenant un groupe hydroxyle et l'oxyde de carbone en présence d'un système catalytique qui comprend :
(a) le palladium et/ou un composé de palladium ;
(b) un ligand de formule I :

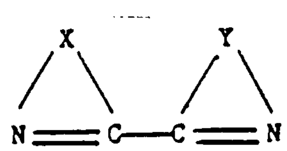

(I)

dans laquelle X et Y représentent des groupes de pontage identiques ou différents dont chacun comporte dans le pontage trois ou quatre atomes dont au moins deux sont des atomes de carbone et ces groupes X et Y peuvent être liés ensemble au moyen d'une liaison autre que celle déjà formée par les atomes de carbone indiquée dans la formule I ; et
(c) un composé MZ dans lequel M est un métal du groupe choisi parmi Cu, Fe, V, Cr, Zn, SN, U et Ce et Z est un anion d'un acide ayant une valeur pK inférieure à 2, à l'exception d'un acide halogenhydrique.

2. Procédé selon la revendication 1, dans lequel le ligand est la 1,10-phénanthroline ou un dérivé de celle-ci.

3. Procédé selon la revendication 1, dans lequel le ligand est le 2,2'-bipyridyle ou un dérivé de celui-ci.

4. Procédé selon les revendications 1 à 3, dans lequel le catalyseur au palladium est un catalyseur homogène.

5. Procédé selon la revendication 4, dans lequel le catalyseur est un sel de palladium.

6. Procédé selon la revendication 5, dans lequel le catalyseur est le diacétate de palladium.

7. Procédé selon les revendications 1 à 6, dans lequel Z est un anion de $H_2SO_4$, $HBF_4$, l'acide p-toluène-sulfonique ou le dérivé alkylsubstitué de celui-ci, ou $HClO_4$.

8. Procédé selon la revendication 7, dans lequel on utilise du tosylate de cuivre(2), du chlorate de cuivre-(2), du chlorate de fer(2), du fluoroborate de cuivre(2), du fluoborate de zinc, du sulfate d'étain(4) ou du sulfate d'uranium(4).

9. Procédé selon l'une quelconque des revendications précédentes, dans lequel on effectue la réaction à une température comprise entre 75 et 200 ° C.

10. Procédé selon l'une quelconque des revendications précédentes, dans lequel on effectue la réaction sous une pression de 10 à 150 bars.

**Patentansprüche**

1. Verfahren zur Herstellung von Carbamaten, umfassend die Umsetzung einer aromatischen Nitroverbindung mit einer Hydroxylgruppen enthaltenden organischen Verbindung und Kohlenmonoxid in Gegenwart eines katalytischen Systems, umfassend
   a) Palladium und/oder eine Palladiumverbindung,
   b) einen Liganden der Formel I

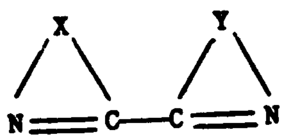

(I)

   in der X und Y gleiche oder verschiedene Brückengruppen bedeuten, von denen jede 3 oder 4 Atome in der Brücke aufweist, von denen mindestens zwei Kohlenstoffatome sind, und wobei die Gruppen X und Y aneinander gebunden sein können über eine andere Bindung als die bereits durch die Kohlenstoffatome, wie in Fig.1 angegeben, gebildete, und
   c) eine Verbindung MZ, in der M ein Metall der Gruppe bestehend aus Cu, Fe, V, Cr, Zn, Sn, U und Ce ist, und Z ein Anion einer Säure mit einem pK-Wert von weniger als 2, mit Ausnahme von Halogenwasserstoffsäure, ist.

2. Verfahren nach Anspruch 1, wobei der Ligand 1,10-Phenanthrolin oder ein Derivat davon ist.

3. Verfahren nach Anspruch 1, wobei der Ligand 2,2'-Bipyridyl oder ein Derivat davon ist.

4. Verfahren nach den Ansprüchen 1 bis 3, wobei der Palladiumkatalysator ein homogener Katalysator ist.

5. Verfahren nach Anspruch 4, wobei der Katalysator ein Palladiumsalz ist.

6. Verfahren nach Anspruch 5, wobei der Katalysator Palladiumdiacetat ist.

7. Verfahren nach den Ansprüchen 1 bis 6, wobei Z ein Anion von $H_2SO_4$ $HBF_4$, p-Toluolsulfonsäure oder einem Alkyl-substituierten Derivat davon, oder $HClO_4$ ist.

8. Verfahren nach Anspruch 7, wobei Kupfer-(II)-tosylat, Kupfer-(II)-chlorat, Eisen-(II)-chlorat, Kupfer-(II)-fluorborat, Zinkfluorborat, Zinn-(IV-sulfat oder Uran-(IV)-sulfat verwendet wird.

9. Verfahren nach einem der vorangehenden Ansprüche, wobei die Reaktion bei einer Temperatur im Bereich von 75 bis 200 ° C durchgeführt wird.

10. Verfahren nach einem der vorangehenden Ansprüche, wobei die Reaktion bei einem Druck im Bereich von 10 bis 150 bar durchgeführt wird.